# EUROPÄISCHE PATENTSCHRIFT

(11) **EP 0 640 344 B1**
(45) Veröffentlichungstag und Bekanntmachung des Hinweises auf die Patenterteilung: **07.10.1998**
(21) Anmeldenummer: 94810472.4
(22) Anmeldetag: 16.08.1994
(51) Int. Cl.: A61K 31/575, A61K 9/00, A61K 47/28

(54) **Ursodeoxycholsäure enthaltendes Arzneimittel in flüssiger Darreichungsform**
Pharmaceutical composition in liquid dosage form containing ursodeoxycholic acid
Composition pharmaceutique sous forme de dosage liquide contenant l'acide ursodésoxycholique

(30) Priorität: 30.08.1993 CH 2567/93
(43) Veröffentlichungstag der Anmeldung: 01.03.1995
(73) Patentinhaber: MEDICHEMIE AG, CH-4107 Ettingen/Basel (CH)
(72) Erfinder: Widauer, Josef Olaf, CH-4123 Allschwil (CH)
(74) Vertreter: Hug Interlizenz AG

(56) Entgegenhaltungen:
- DE-A- 1 904 562
- PATENT ABSTRACTS OF JAPAN vol. 011, no. 394 (C-465) 23. Dezember 1987 & JP-A-62 153 220 (TOKYO TANABE CO.LTD.) 8. Juli 1987
- PATENT ABSTRACTS OF JAPAN vol. 004, no. 050 (C-007) 16. April 1980 & JP-A-55 022 616 (TOKYO TANABE CO.LTD.) 18. Februar 1980

## Beschreibung

### TECHNISCHES GEBIET

Die vorliegende Erfindung betrifft ein Ursodeoxycholsäure enthaltendes Arzneimittel in flüssiger Darreichungsform insbesondere zu Behandlung cholestatischer Lebererkrankungen bei Kindern.

Im menschlichen Gallenpool sind verschiedene Gallensäuren vorhanden, darunter normalerweise zu 70-80% Chenodesoxycholsäure und zu ca. 3% die erwähnte Ursodesoxycholsäure. Beim Cholestasesyndrom kommt es wegen eines zu geringen oder gar fehlenden Abflusses von Galle in den Darm zu einem Rückstau von Gallenflüssigkeit in die Leber und einer Schädigung von Leberzellen vor allem durch die aggresive Chenodesoxycholsäure. Durch gezielte Gabe von Ursodesoxycholsäure lässt sich das Verhältnis der beiden Gallensäuren im Gallensäurepool jedoch stark zugunsten der wesentlich weniger aggresiven Ursodesoxycholsäure verändern und dadurch die Aggressivität der Gallenflüssigkeit insgesamt erheblich reduzieren.

### STAND DER TECHNIK

Alle Gallensäuren, so auch die Ursodesoxycholsäure, weisen einen extrem bitteren Geschmack und einen ebenso bitteren, über Stunden anhaltenden Nachgeschmack auf. Bei der gängigen oralen Darreichung in Form von Kapseln oder Tabletten kann der bittere Geschmack zwar wirksam kaschiert werden, doch sind diese Darreichungsformen insbesondere in der Pädiatrie kaum anwendbar, da Kapseln oder Tabletten von Kindern nicht oder nur unter Schwierigkeiten geschluckt werden. In der Pädiatrie werden deshalb grundsätzlich flüssige Darreichungsformen bevorzugt, zumal sich diese bei Kindern abgestimmt auf deren Körpergewicht besser dosieren lassen. Auch bei flüssigen Darreichungsformen sind Geschmackskaschierungen möglich, z.B. durch Verwendung sogenannter Pellets. Pellets sind kleine Kügelchen, in den der Wirkstoff eingeschlossen und so dem direkten Kontakt mit der Mundschleimhaut entzogen ist. Die Pellets werden in Suspensionen dispergiert verabreicht. Die Herstellung der Pellets ist allerdings aufwendig und teuer. Sie sind sehr fragil, so dass die Gefahr besteht, dass sie zerbrechen oder zerbissen werden. Zudem lassen sich in der Regel nur relativ niedrige Wirkstoffmengen pro Gewichtseinheit unterbringen. Im Falle der Ursodesoxycholsäure ist auf diese Weise eine Konzentration von allenfalls 20-30 mg/ml erreichbar, wohingegen eine solche von ca. 50 mg/ml erwünscht wäre. Um die Ursodesoxycholsäure in genügend hoher Dosierung (15-20mg/kg-Kindergewicht und Tag) verabreichen zu können, hat man deshalb bis heute auf eine Geschmackskaschierung praktisch verzichtet, eine Lösung der Gallensäure (in Natriumbikarbonat) hergestellt und diese per Sonde verabreicht.

### DARSTELLUNG DER ERFINDUNG

Die vorliegende Erfindung stellt sich die Aufgabe eine geschmacklich akzeptable flüssige Darreichungsform für die Ursodesoxycholsäure mit einer ausreichend hohen Wirkstoffkonzentration anzugeben. Diese Aufgabe wird erfindungsgemäss dadurch gelöst, dass die einzunehmende Flüssigkeit eine unter Zugabe eines Quell- bzw. Verdickungsmittels hergestellte Suspension ist, welche den Wirkstoff überwiegend in feinkristalliner Form als disperse Phase und nur zu einem wesentlich geringeren Anteil im wässrigen Dispersionmittel gelöst enthält.

Die durch die Erfindung angegebene Formulierung der Ursodesoxycholsäure weist eine vergleichsweise nur noch sehr geringe Restbitterkeit auf. Die starke Reduktion des extrem bitteren Geschmacks der Gallensäure ist vermutlich darauf zurückzuführen, dass die feinen Säurekristalle (Korngrösse vorzugsweise zu über 99% < 90µm) von dem Quell- bzw. Verdikkungsmittel einzeln eingeschlossen und ummantelt werden. Beim Einnehmen der Flüssigkeit können die Säurekristalle dadurch mit der Mundschleimhaut nicht oder nur zu einem geringen Anteil in direkten Kontakt treten. Obwohl derartige Geschmackskaschierungen an sich bekannt sind, ist das Ausmass, in dem die Bitterkeit der Gallensäure im vorliegenden Fall dadurch reduzierbar ist, überraschend und unerwartet. Die noch vorhandene Restbitternis dürfte vor allem durch den im Dispersionsmittel in Lösung befindlichen Wirkstoffanteil verursacht werden. Die Einbettung der Säurekristalle im Quell-bzw. Verdickungsmittel wirkt sich auch dahingehend günstig aus, als sie deren Lösung im Dispersionsmittel erschwert. Bei 50 mg/ml Wirkstoff beträgt der gelöste Wirkstoffanteil so nur etwa ein halbes Prozent.

Das Dispersionsmittel ist vorzugsweise einfach demineralisiertes Wasser. Als Quell- bzw. Verdickungsmittel kommen verschiedene Substanzen in Frage, darunter z.B. das Aluminiumsilikat Bentonit oder verschiedene Formen von Methylcellulose, Hydroxypropylmethylcellulose, Hydroxypropylcellulose, Hydroxyethylcellulose und Gallactomannane. Im Hinblick auf die Langzeitstabilität der Suspension sowie deren Fliesseigenschaften (Viskosität, Thixotropie) hat sich als besonders geeignet eine in Wasser dispergierbare mikrokristalline Cellulose erwiesen, die unter der Markenbezeichnung AVICEL® RC 591 von der Firma FMC Corporation, Philadelphia, USA, angeboten wird. Diese enthält als Schutzkolloid einen Anteil Natriumcarboxymethylcellulose. Andere von der gleichen Firma unter der Bezeichnung AVICEL® angebotene Quellmittel sind ebenfalls grundsätzlich verwendbar.

Die für die Praxis besonders geeignete Wirkstoffkonzentration beträgt 50 mg/ml. Suspensionen mit eventuell benötigten anderen Wirkstoffzentrationen (z.B. lediglich 25 mg/ml oder auch bis zu 75 mg/ml) sind jedoch ebenfalls herstellbar.

Die durch den gelösten Wirkstoffanteil erzeugte Restbitternis lässt sich weiter reduzieren durch Zugabe von β-Cyclodextrin. β-Cyclodextrin ist ein ringförmiges Molekül, aufgebaut aus sieben Glucose-Einheiten. Das Ringinnere ist hydrophob und befähigt, ein hydrophobes Molekül zu binden. Das Ringäussere ist hydrophil und verträgt sich mit dem wässrigen Dispersionsmittel. Das Cyclodextrin bildet mit dem gelösten Wirkstoff eine Einschlussverbindung, indem das Wirkstoffmolekül als "Gast" im Cyclodextrin-"Gastgeber"molekül eingeschlossen wird. Es kommt dadurch wiederum zu einer räumlichen Abschirmung des Wirkstoffmoleküls.

Die erfindungsgemässe Suspension kann schliesslich noch mit Konservierungs- und zur weiteren Geschmacksverbesserung mit Süssungsmitteln sowie Aromatica und Natriumchlorid versetzt sein.

Der pH-Wert spielt ebenfalls bezüglich der Bitternis eine wichtige Rolle. Der Wirkstoff geht mit steigendem pH unter Salzbildung in Lösung und verstärkt dadurch die Bitternis. Deshalb ist es von Vorteil, durch geeignete Puffersysteme (z.B. Phosphat- oder Citrat-Puffer, z.B. McIlvaine-Puffer) den pH-Wert der Suspension zwischen 2,5 und 8, bevorzugt 3,5 bis 6,0, einzustellen.

### BEISPIELE:

Nachstehend ist eine bevorzugte Zusammensetzung für 1 ml der erfindungsgemässen Suspension angegeben. Die Werte in Klammern geben jeweils den möglichen Mengen-Variationsbereich der einzelnen Bestandteile an, wobei die jeweils kleinsten Werte bei der angegeben kleinsten Wirkstoffmenge und die jeweils grössten Werte bei der angegeben grössten Wirkstoffmenge zur Anwendung kommen.

| **Komponente** | **Menge (g)** | **Funktion** |
|---|---|---|
| Ursodesoxyxholsäure fein | 0.05 (0.025-0.075) | Wirkstoff |
| β-Cyclodextrin | 0.1 (0.015-0.3) | Geschmacksmaskierer |
| AVICEL®RC591 | 0.01 (0.005-0.03) | Verdickungsmittel |
| Saccharose | 0.3 (0.15-0.45) | Süssungsmittel |
| Methylparaben | 0.0013 (0.0012-0.0014) | Konservierungsmittel |
| Propylparaben | 0.0002 (0.0001-0.0003) | Konservierungsmittel |
| Propylenglykol | 0.05 (0.045-0.055) | Lösungsvermittler für Konservierungsmittel |
| Aromatica | 0.0013 (0,001-0,0015) | |
| Demineralisiertes Wasser | ad 1.00 ml | Dispersionsmittel |

oder zuckerfreie Form:

| **Komponente** | **Menge (g)** | **Funktion** |
|---|---|---|
| Ursodesoxycholsäure fein | 0.05 | Wirkstoff |
| Hydroxyethylcellulose | 0.008 (0.004-0.012) | Verdickungsmittel |
| Benzoesäure | 0.0015 | Konservierungsmittel |
| Xylitol | 0.32 (0.25-0.4) | Süssungsmittel |
| Glycerin | 0.1 (0.05-0.2) | Süssungsmittel |
| Natrii cyclamas | 0.005 | Süssungsmittel |
| Natrii chloridum | 0.0007 (0.0005-0.002) | Geschmacksmaskierer |
| Acidum citricum | 0.002 | Puffer |
| Natrii citras | 0.0014 | Puffer |
| Aroma | 0.0005 | |
| Aqua purificata | ad 1.0 (ml) | |

Die Herstellung einer Suspension in einer der vorstehend angegebenen Zusammensetzungen erfolgt vorzugsweise in folgenden Schritten:
- Methyl- und Propylparaben werden in einem Gefäss im Propylenglykol unter Rühren bei ca. 70°C vollständig gelöst und abkühlen gelassen;
- Das Wasser wird in einem Gefäss vorgelegt. Das AVICEL®RC 591 wird mit einem hochtourigen Homogenisierstab darin dispergiert.
- Nacheinander werden der Wirkstoff Ursodesoxycholsäure und das β-Cyclodextrin in Portionen mittels Homogenisierstab darin eingearbeitet;
- Die Parabenlösung wird zugegeben;
- Die Saccharose wird mittels Homogenisierstab eingearbeitet;
- Nach dem Ruhenlassen von mehreren Stunden wird das Aroma zugegeben und die entstandene Suspension nochmals kurz homogenisiert.

Die Herstellung einer erfindungsgemässen Suspension ohne β-Cyclodextrin erfolgt vorzugsweise wie folgt:
- Methyl- und Propylparaben werden in einem Gefäss im Propylenglykol unter Rühren bei ca. 70°C vollständig gelöst und abkühlen gelassen;
- In 2/3 des Wassers wird die Saccharose in einem Gefäss unter Rühren bei Raumtemperatur gelöst;
- In 1/3 des Wassers wird das AVICEL®RC 591 in einem Gefäss mit einem hochtourigen Homogenisierstab eingearbeitet; Die Parabenlösung und die Saccharoselösung werden zur AVICEL® RC 591-Disperion gegeben;
- Der Wirkstoff Ursodesoxycholsäure wird in mehreren Portionen mittels Homogenisierstab eingearbeitet;
- Das Aroma wird zugegeben und die entstandene Suspension nochmals kurz homogenisiert.

Die Herstellung einer erfindungsgemässen Suspension ohne Zucker erfolgt vorzugsweise wie folgt:
- Hydroxyethylcellulose wird in 2/3 Wasser unter Rühren bei ca 40°C gelöst;
- Benzoesäure, Xylitol, Natriumcyclamat, Natriumchlorid, Citronensäure und Natriumcitrat werden in der Hydroxyethylcellulose-Lösung gelöst;
- Glycerin wird mit 1/3 Wasser gemischt. Darin wird Ursodesoxycholsäure mittels Homogenisierstab bei Raumtemperatur eingearbeitet;
- Die Ursodesoxycholsäure-Suspension wird in der Hydroxyethylcellulose-Lösung unter Rühren eingearbeitet;
- Das Aroma wird unter Rühren zugegeben.

## Patentansprüche

1. Arzneimittel in flüssiger Darreichungsform enthaltend Ursodeoxycholsäure als Wirkstoff insbesondere zur Anwendung bei der Behandlung cholestatischer Lebererkrankungen bei Kindern, dadurch gekennzeichnet, dass die einzunehmende Flüssigkeit eine unter Zugabe eines Quell- bzw. Verdickungsmittels hergestellte Suspension ist, welche den Wirkstoff überwiegend in feinkristalliner Form als disperse Phase und nur zu einem wesentlich geringeren Anteil im wässrigen Dispersionsmittel gelöst enthält.

2. Arzneimittel nach Anspruch 1, dadurch gekennzeichnet, dass über 99%, vorzugsweise 99,4%, des feinkristallinen Wirkstoffes eine Kerngrösse kleiner als 90 µm aufweist.

3. Arzneimittel nach einem der Ansprüche 1 oder 2, dadurch gekennzeichnet, dass als Dispersionsmittel demineralisiertes Wasser verwendet ist.

4. Arzneimittel nach einem der Ansprüche 1 bis 3, dadurch gekennzeichnet, dass das Quell- bzw. Verdickungsmittel eine in Wasser dispergierbare mikrokristalline Cellulose ist, die einen Anteil Natriumcarboxymethylcellulose als Schutzkolloid enthält und in einem Mengenbereich bon 0,005-0,03 g/ml liegt.

5. Arzneimittel nach einem der Ansprüche 1 bis 4, dadurch gekennzeichnet, dass die Suspension zusätzlich β-Cyclodextrin als Geschmacksmaskierer für im Dispersionmittel gelösten Wirkstoff enthält.

6. Arzneimittel nach einem der Ansprüche 1 bis 5, dadurch gekennzeichnet, dass die Suspension zusätzlich Methylparaben und/oder Propylparaben und/oder Benzoesäure und/oder Sorbinsäure als Konservierungsmittel enthält.

7. Arzneimittel nach Anspruch 6, dadurch gekennzeichnet, dass die Suspension zusätzlich Propylenglykol als Lösungsvermittler für das/die Konservierungsmittel enthält.

8. Arzneimittel nach einem der Ansprüche 1 bis 7, dadurch gekennzeichnet, dass die Suspension zusätzlich Saccharose als Süssungsmittel und/oder Aromastoffe enthält.

9. Arzneimittel nach einem der Ansprüche 1 bis 7, dadurch gekennzeichnet, dass die Suspension Zuckeraustauschstoffe wie Sorbit, Mannit, Xylit und/oder Süssstoffe wie Saccharin, Cyclamat-Natrium, Aspartam oder Acesulfam-Kalium enthält

10. Arzneimittel nach einem der Ansprüche 1 bis 9, dadurch gekennzeichnet, dass der pH-Wert der Suspension auf einen Wert zwischen 2,5 und 8, bevorzugt 3,5 bis 6,0, eingestellt ist

## Claims

1. Medicament in a liquid administration form containing ursodeoxycholic acid as the active agent, particularly for the treatment of cholestatic hepatic diseases in infants, characterized in that the liquid to be taken is a suspension prepared accompanied by the addition of a swelling and/or thickening agent, which contains the active agent mainly in a finely crystalline form as the disperse phase and only in a much smaller proportion dissolved in the aqueous dispersant.

2. Medicament according to claim 1, characterized in that over 99%, preferably 99.4%, of the fine crystalline active agent has a nucleus size smaller than 90 µm.

3. Medicament according to either of the claims 1 and 2, characterized in that demineralized water is used as the dispersant.

4. Medicament according to any one of the claims 1 to 3, characterized in that the swelling or thickening agent is a microcrystalline cellulose, which is dispersible in water and which contains a proportion of sodium carboxymethyl cellulose as the protective colloid and is present in a quantity range of 0.005 to 0.03 g/ml.

5. Medicament according to any one of the claims 1 to 4, characterized in that the suspension additionally contains β-cyclodextrin as the taste masking agent for the active agent dissolved in the dispersant.

6. Medicament according to any one of the claims 1 to 5, characterized in that the suspension additionally contains as preservatives methylparaben and/or propylparaben and/or benzoic acid and/or sorbic acid.

7. Medicament according to claim 6, characterized in that the suspension additionally contains propylene glycol as the solubilizer for the preservative or preservatives.

8. Medicament according to any one of the claims 1 to 7, characterized in that the suspension additionally contains saccharose as a sweetener and/or flavouring agents.

9. Medicament according to any one of the claims 1 to 7, characterized in that the suspension contains sugar substitutes such as sorbitol, mannitol, xylitol and/or sweeteners such as saccharin, sodium cyclamate, aspartame or potassium acesulphame.

10. Medicament according to any one of the claims 1 to 9, characterized in that the pH-value of the suspension is adjusted to between 2.5 and 8, preferably between 3.5 and 6.

## Revendications

1. Médicament sous forme galénique liquide, contenant de l'acide ursodésoxycholique, en tant que principe actif, destiné en particulier à être utilisé dans le traitement de maladies cholestatiques du foie chez l'enfant, caractérisé par le fait que le liquide à absorber est une suspension préparée par addition d'un agent gonflant ou épaississant qui contient le principe actif principalement sous forme finement cristallisée, en tant que phase dispersée, et seulement en une proportion nettement inférieure sous forme dissoute, dans un milieu de dispersion aqueux.

2. Médicament selon la revendication 1, caractérisé par le fait que plus de 99 %, de préférence plus de 99,4 %, du principe actif finement cristallisé présente une grosseur de grains inférieure à 9,0 µm.

3. Médicament selon l'une des revendications 1 ou 2, caractérisé par le fait que l'on utilise de l'eau déminéralisée en tant que milieu de dispersion.

4. Médicament selon l'une quelconque des revendications 1 à 3, caractérisé par le fait que l'agent gonflant ou épaississant est une cellulose microcristalline dispersible dans l'eau, qui contient une proportion de carboxyméthylcellulose sodique en tant que colloïde protecteur et qui est présente en une proportion de 0,005-0,03 g/ml.

5. Médicament selon l'une quelconque des revendications 1 à 4, caractérisé par le fait que la suspension contient en outre de la β-cyclodextrine pour masquer le goût du principe actif dissous dans l'agent dispersant.

6. Médicament selon l'une quelconque des revendications 1 a 5, caractérisé par le fait que la suspension contient en outre du méthylparaben et/ou du propylparaben et/ou de l'acide benzoïque et/ou de l'acide sorbique, en tant qu'agent conservateur.

7. Médicament selon la revendication 6, caractérisé par le fait que la suspension contient en outre du propylèneglycol en tant que solubilisant du/des agent(s) conservateur(s).

8. Médicament selon l'une quelconque des revendications 1 à 7, caractérisé par le fait que la suspension contient en outre du saccharose en tant qu'édulcorant et/ou des substances aromatisantes.

9. Médicament selon l'une quelconque des revendications 1 à 7, caractérisé par le fait que la suspension contient des succédanés de sucre tels que le sorbite, le mannite, le xylite et/ou des édulcorants tels que la saccharine, le cyclamate de sodium, l'aspartam ou l'acésulfame de potassium.

10. Médicament selon l'une quelconque des revendications 1 à 9, caractérisé par le fait que le pH de la suspension est ajusté à une valeur comprise entre 2,5 et 8, de préférence entre 3,5 et 6,0.
